# EUROPEAN PATENT APPLICATION

(11) **EP 4 050 614 A1**
(43) Date of publication of application: **31.08.2022**
(21) Application number: 21159589.7
(22) Date of filing: 26.02.2021
(51) Int. Cl.: G16H 10/40

(54) **METHOD AND SYSTEM OF MANAGING SAMPLE PRIORITIES**

(71) Applicant: F. Hoffmann-La Roche AG, 4070 Basel (CH); Roche Diagnostics GmbH, 68305 Mannheim (DE); Hitachi High-Tech Corporation, Minato-ku Tokyo 105-6409 (JP)
(72) Inventor: The designation of the inventor has not yet been filed
(74) Representative: Altmann Stößel Dick Patentanwälte PartG mbB

(57) **Abstract**

The present disclosure relates to a computer implemented method of managing sample processing priorities in a diagnostic laboratory comprising at least one sample processing device (100) connected to a host system (200). The method comprises generating a communication message (20) between the host system (200) and the at least one sample processing device (100) related to a sample processing order (10) received in association with a sample, the message (20) comprising one of at least two priority identifiers (R, S) indicative of respective sample processing priorities from lower priority to higher priority according to the received sample processing order (10). The method further comprises identifying (30) the sample by the at least one sample processing device (100) and processing the sample (31, 32) by the at least one sample processing device (100) according to the sample processing priority (S, R) in the message (20). In case of receiving a subsequent request for change (40) of sample processing priority for the sample from a lower priority to a higher priority after transmission of the message (20) and before the sample is processed, the method comprises changing (50) the message (20) and processing the sample as a higher priority sample (31) instead of as a lower priority sample (32), wherein changing the message (20) comprises changing the lower priority identifier (R) to an identifier (CS) indicative of a change of priority but different from an equivalent higher priority identifier (S) in order to maintain the sample and the sample processing order uniquely identifiable and traceable by both the host system (200) and the at least one sample processing device (100). A respective system for managing sample processing priorities is herein also disclosed.

## Description

### Technical Field

The present disclosure relates to a computer-implemented method and to a system for managing sample processing priorities in a diagnostic laboratory.

### Background

In diagnostic laboratories, in particular clinical laboratories, a multitude of analytical tests on patient samples are routinely executed by automatic sample processing devices in order to obtain information about medical patient conditions. For some patients in critical care, obtaining such information as quick as possible may be very important and in some cases of life-critical importance. Analytical tests ordered by physicians, at least for the larger laboratories and/or care institutions, are typically registered centrally in a host system such as a laboratory information system (LIS) and/or hospital information system (HIS) in communication with a diagnostic laboratory and with laboratory sample processing devices. All data relevant for sample identification (ID) and processing can be registered for each sample in the host system and communicated to the target sample processing device(s) so that each sample can be traced, thus the correct ordered sample processing workflow or tests can be executed, and the sample processing results can be uniquely associated with each sample and hence with the patient the sample came from.

According to current practice, samples, respectively sample processing orders which need urgent processing are marked as urgent or as short turnaround time (STAT) samples. STAT samples and related STAT sample processing orders are given higher sample processing priority with respect to routine samples and related routine sample processing orders. The sample processing priority, either routine or STAT, is typically part of the registered data associated with a sample ID, typically together with other data, such as the sample type, a list of analytical tests to be made with the sample and other sample processing instructions.

Sometimes there is a need to change the sample processing priority for a sample that has already been registered as a routine sample, from routine sample to STAT sample, at a time sample processing has not yet started. This case may arise for example when a patient condition suddenly worsens and it becomes important to obtain the sample processing results earlier, or because obtaining a sample processing result earlier than originally expected may play an important role in patients management or for any other reason, or simply because the initial sample processing priority was wrongly assigned or the sample urgency wrongly determined.

However, it is typically not possible or not allowed to change the sample processing priority after registration since conflicts and problems with unique sample identification and traceability may arise, e.g. causing a mismatch between host and sample processing device, or causing confusion between two different samples, where the only difference associated with respective sample identities prior to the change was e.g. in the sample processing priority.

### General Description

In view of the above background, a computer-implemented method and a system for managing sample processing priorities in a diagnostic laboratory are herein disclosed that enable to change the sample processing priority from lower priority to higher priority, following an initial request for sample processing and an initial sample registration with lower sample processing priority, before the sample is processed, and thus to process the sample as a higher priority sample, while maintaining the sample uniquely identifiable and traceable. Other advantages will appear from the following description.

In particular, a computer-implemented method of managing sample processing priorities in a diagnostic laboratory comprising at least one sample processing device connected to a host system is disclosed. The method comprises generating a communication message between the host system and the at least one sample processing device related to a sample processing order received in association with a sample, the message comprising one of at least two priority identifiers indicative of respective sample processing priorities from lower priority to higher priority according to the received sample processing order. The method further comprises identifying the sample by the at least one sample processing device and processing the sample by the at least one sample processing device according to the sample processing priority in the message. In case of receiving a subsequent request for change of sample processing priority for the sample from a lower priority to a higher priority after transmission of the message and before the sample is processed, the method comprises changing the message and processing the sample as a higher priority sample instead of as a lower priority sample, where changing the message comprises changing the lower priority identifier to an identifier indicative of a change of priority but different from an equivalent higher priority identifier in order to maintain the sample and the sample processing order uniquely identifiable and traceable by both the host system and the at least one sample processing device.

A "sample processing device" can be any laboratory device either as a stand-alone apparatus or module within a larger connected system configured to execute sample processing orders for in-vitro-diagnostics. "Sample processing" means either detection, e.g. qualitative and/or quantitative evaluation of samples for diagnostic purpose, and/or preparation of samples before detection, or storing and/or disposal of samples after detection. In particular, a lab device may be related to analytical and/or to pre-analytical and/or to post-analytical sample processing steps. Sample processing devices may be connected to each other and depend at least in part on each other, e.g. each carrying out a dedicated task of a sample processing workflow, which may be a prerequisite before proceeding to the next sample processing device. Alternatively, sample processing devices may work independently from each other, e.g. each carrying out a separate task, e.g. a different type of analysis on the same sample or different sample.

An "analytical sample processing device" is a sample processing device for e.g. qualitative and/or quantitative evaluation of samples for diagnostic purpose. It may comprise a detection system and be configured to execute a workflow optimized for certain types of analysis. Examples of such analytical sample processing devices are clinical chemistry analyzers, coagulation chemistry analyzers, immunochemistry analyzers, urine analyzers, hematology analyzers, molecular diagnostics analyzers, mass spectrometry analyzers, and the like (the list is not exhaustive) used to detect analytes in a sample or other sample parameters, typically as a result of chemical or biological reactions involving treating of samples with reagents. An analytical sample processing device can comprise functional units such as liquid handling units for pipetting and/or pumping and/or mixing of samples and/or reagents and/or system fluids, and also functional units for sorting, storing, transporting, identifying, separating, detecting. The analytical sample processing device may comprise a reagent holding unit for holding reagents to perform the assays. Reagents may be arranged for example in the form of containers or cassettes containing individual reagents or group of reagents, placed in appropriate receptacles or positions within a storage compartment or conveyor, e.g. a rotor. It may comprise a reaction vessel or cuvette holding unit. In particular, it may comprise one or more liquid processing units, such as a pipetting unit, to deliver samples and/or reagents to the reaction vessels. The pipetting unit may comprise a reusable washable needle, e.g. a steel needle, or disposable pipette tips. The analytical sample processing device may further comprise one or more mixing units, comprising e.g. a shaker to shake a cuvette comprising a liquid or a mixing paddle or stirrer to mix liquids in a cuvette or reagent container.

A "pre-analytical sample processing device" can be either a stand-alone apparatus or module within a larger connected system typically configured for sorting and/or for checking sample quality and/or for preparation of samples before being further processed by an analytical sample processing device, possibly including process steps like reformatting, aliquoting and the like. It may comprise for example one or more of the following: a resorting unit to sort samples according to type of analysis and/or sample processing priority, a centrifuge for centrifuging sample tubes, an aliquoting unit where a pipetting unit is used to aliquot samples from sample tubes, a thermal treatment unit to subject the sample to a certain temperature, a separation unit to separate sample components, and the like.

A "post-analytical sample processing device" is either a stand-alone apparatus or module within a larger connected system typically configured e.g. for storing and/or disposal of samples after being processed by an analytical work cell. It may comprise for example a resorting or reformatting unit to resort sample tubes, e.g. to different storage racks, and/or a refrigerated compartment.

The term "sample processing device" may further include sample transportation devices or modules configured to transport samples between other lab devices, e.g. comprising conveyors either mechanical or based on other principles such as induction, to move samples, typically in sample tubes, on single-tube carriers or multi-tube carriers such as racks.

The term "sample processing device" may further include a robotic device programmed to interact with one or more other lab devices, e.g. to supply samples and/or consumables to an analytical lab device or to perform maintenance tasks, in an automated manner.

Sample processing devices may have different configurations according to the need and/or according to the desired laboratory workflow. Additional configurations may be obtained by coupling a plurality of apparatuses and/or modules together, where each module may have a dedicated function. Thus each laboratory may be configured in a different manner according to user requirements and needs, e.g. in terms of sample processing throughput, type of analysis, type of samples and the like.

The term "sample" may refer to any biological material derived from a patient that may potentially contain one or more analytes of interest, whose determination by testing may be indicative of a patient condition. The sample can be derived from any biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cultured cells, or the like. The sample can be pretreated prior to use, such as preparing plasma or serum from blood, diluting viscous fluids, lysing or the like. Methods of treatment can involve filtration, distillation, concentration, inactivation of interfering components, and the addition of reagents. A sample may be used directly as obtained from the source in some cases or following a pretreatment and/or sample preparation workflow to modify the character of the sample, e.g., after adding an internal standard, after being diluted with another solution or after having been mixed with reagents, e.g., to enable carrying out one or more in vitro diagnostic tests, or for enriching (extracting/separating/concentrating) analytes of interest and/or for removing matrix components potentially interfering with the detection of the analyte(s) of interest. Samples may be provided for example in sample containers such as sample tubes, including primary tubes and secondary tubes, or multi-well plates, or any other sample carrying support, open or closed.

The term "sample processing order" as used herein refers to a request, typically initiated by a healthcare practitioner, of a particular process to be executed on a particular patient sample by at least one sample processing device, typically in expectation of a returning sample processing result within a certain time. A sample processing order typically includes an order for an analytical test, but can refer in addition or in alternative also to pre- and/or post-analytical processing steps to be performed on the sample.

The term "sample processing result" as used herein may encompass any data that is descriptive of a result of a sample processing order and can be in particular related to an analytical test result, that is the determination of analyte(s) in a sample, namely qualitative and/or quantitative measurement of analyte(s) in a sample, or the determination of other sample parameter(s). The term "sample processing result" may also encompass calculated results based on measurement of one or more analytes in a sample, as risk scores or other values calculated using various clinical decision support algorithms.

Since the sample processing devices have a predefined maximum sample processing throughput and limited capacity, it is important to prioritize sample processing. Samples are thus typically categorized according to their sample processing priority as either STAT samples or routine samples.

A "short-turnaround-time (STAT) sample" is a sample which needs to be processed more urgently, i.e. with higher priority, than other standard or routine samples because the respective sample processing result is needed more urgently. There may be various possible reasons why sample processing results are needed as soon as possible, e.g. in order to manage medical emergencies or because of the sample type or sample processing type, or e.g. because of sample or analyte deterioration within the time it would take to process the sample as a routine sample. In such cases the samples are categorized as STAT samples. Such categorization may be manual, automatic or semiautomatic, e.g. based on a set of rules that may be implemented e.g. at the host and/or executed by the host, e.g. based on sample type and/or analytical test type, sample processing device availability, and the like.

The term "host" is herein used to intend a central management system or middleware layer for management of patient and/or laboratory information, in communication with one or more sample processing devices, and typically comprising a server or host computer, a database and a database management system, and application software or user interface for entering and processing patient and/or laboratory information, in particular for centrally registering patient samples and sample processing orders related to the patient samples, but possibly also for sample / sample processing order tracking, quality control, compliance, inventory management and maintenance, and for receiving and managing of sample processing results. The term "host" may particularly include a Laboratory Information Systems (LIS) and/or a Hospital Information System (HIS), and possibly in addition any one or more of a dedicated inventory management system, a maintenance management, an operator management system and the like.

The communication between host and sample processing devices can be unidirectional or bidirectional and takes place in the form of "messages" sent in one or both directions between the host and one or more sample processing devices, comprising acknowledgment message pairs.

The term "communication message" or "message" for short, as used herein, refers particularly to a group of segments in a defined sequence comprising strings of codes or identifiers, using a syntax compatible with the communication protocol used, that can define different message types according to their purpose. The message can be based on protocols typically used in the lab device and healthcare industry like the standard Health Level Seven (HL7) or Fast Healthcare Interoperability Resources (FHIR), which can be adapted in a proprietary manner specific for a proprietary host-lab device communication. Other protocols, such as the IEEE 1073 Standard for Medical Device Communication or proprietary protocols designed for medical device communications can be implemented as well.

According to an embodiment, the message is in an HL7 or FHIR format.

In particular, a message, that is generated, typically by the host, upon entering information related to a sample and to a sample processing order, may comprise several segments and respective codes or identifiers within the segments required for sample processing and/or reporting of the sample processing result. Thus, for example, there may be various segments and identifiers within the segments e.g. related to patient ID and patient data, sample ID, the type of sample, the type of sample container, the type of sample carrier, the target sample processing device(s), the particular analytical test(s) to be executed with the sample, the availability of analytical tests, e.g. based on actual availability of consumables required to execute the analytical test(s), a sequence of workflow steps or instructions for executing the sample processing, e.g. specific to the selected analytical test(s), and in particular identifiers indicative of sample processing priority. The list is not exhaustive. In addition, messages may comprise different combinations of segments and identifiers and in a different order.

According to the present disclosure, the message comprises one of at least two priority identifiers indicative of respective sample processing priorities from lower priority to higher priority according to the received sample processing order. For example, although different degrees of sample processing priority can be assigned to a sample, e.g. more than two, e.g. from 1 to 3 or 1 to 5 or 1 to 10, with 1 being indicative of the highest priority, the message, when generated for the first time with respect to a sample processing order, comprises an identifier indicative at least of either a routine sample with lower sample processing priority or a STAT sample with higher sample processing priority.

The method further comprises identifying the sample by the at least one sample processing device and processing the sample by the at least one sample processing device according to the sample processing priority defined in the message. Identifying the sample by the at least one sample processing device may comprise for example reading a barcode, an RFID label or other type of label, either on the sample container and/or sample container carrier.

In case of receiving a subsequent request for change of sample processing priority for the sample from a lower priority to a higher priority, e.g. a request for change from a routine sample to a STAT sample, after transmission of the message and before the sample is processed, the method comprises changing the message and processing the sample as a higher priority sample, e.g. as a STAT sample, instead of as a lower priority sample, e.g. as a routine sample. Thus a routine sample, upon arrival and identification by the at least one sample processing device, or even after arrival and identification, e.g. when arriving to an analytical sample processing device after initial process steps as a lower priority sample in a pre-analytical sample processing device, or e.g. when it is in a queue, waiting in line for its turn according to a scheduled plan, e.g. in a sample buffer, together with other routine samples, may be re-categorized as a STAT sample and given the right and order to skip the line and be processed earlier than initially scheduled and before other routine samples. However, changing the message comprises changing the lower priority identifier to an identifier indicative of a change of priority but different from an equivalent higher priority identifier. For example, changing the message comprises changing the identifier used to indicate a routine sample to an identifier indicative of a change from a routine sample to a STAT sample but different from the identifier used to indicate a STAT sample. In this way, it is possible to maintain the sample and the sample processing order uniquely identifiable and traceable by both the host system and the at least one sample processing device.

According to an embodiment, changing the message comprises providing a user interface, typically at the host system, with an option to select the sample or a sample carrier to be processed with higher priority, and an option to select a change of priority command for the selected sample or sample carrier.

The term "user interface" as used herein refers to a hardware and software layer providing a user with the function and option to interact with the host and/or with the at least one sample processing device, in order e.g. to enter sample and/or sample processing orders, to provide instructions to the at least one sample processing device, to monitor the status of sample processing orders, to review sample processing results, to monitoring the operational status and/or inventory status of the at least one sample processing device, and in particular to change the sample processing priority, as mentioned above. The user interface may be any sort of user interface like graphical user interface, command line interface, menu-driven user interface, touch user interface, voice user interface, form-based user interface, virtual reality user interface and can include the use of a display screen, a keyboard, a mouse, a computing device such as a desktop, laptop, tablet, smartphone or wearable device like a smartwatch or virtual reality devices, e.g. glasses configured to facilitate and enhance user experience.

According to an embodiment, the method comprises highlighting or marking the selected sample and/or sample processing order and/or sample processing result related to the selected sample in the user interface after a change of priority such as to be distinguishable in a list of samples and/or of sample processing orders and/or sample processing results.

According to an embodiment, a change of sample processing priority is allowed only for routine samples resulting in a change from a routine sample with lower priority to a STAT sample with higher priority, regardless of type of sample carrier. In this case, the option to select a change of priority for e.g. controls and calibrators is excluded. In other words, a change of priority may be allowed only for some routine samples in case there is an effective need to do so, whereas any other changes to a schedule of sample processing and quality control procedures may not be allowed.

A system for managing sample processing priorities in a diagnostic laboratory comprising at least one sample processing device connected to a host system is herein further disclosed. The system is configured for generating a message between the host system and the at least one sample processing device related to a sample processing order received in association with a sample, the message comprising one of at least two priority identifiers indicative of respective sample processing priorities from lower priority to higher priority according to the received sample processing order. The at least one sample processing device is configured for identifying the sample and processing the sample according to the sample processing priority in the message. The system is further configured for receiving a subsequent request for change of sample processing priority for the sample from a lower priority to a higher priority after transmission of the message and before the sample is processed, comprising changing the message and processing the sample as a higher priority sample instead of as a lower priority sample, where changing the message comprises changing the lower priority identifier to an identifier indicative of a change of priority but different from an equivalent higher priority identifier in order to maintain the sample and the sample processing order uniquely identifiable and traceable by both the host system and the at least one sample processing device.

The term "system for managing sample processing priorities" as used herein may include any physical or virtual processing device and in particular a programmable logic controller running a computer-readable program provided with instructions to perform operations in accordance with an operation plan. This may include a processor, a controller, a central processing unit (CPU), a microprocessor, a microcontroller, a reduced instruction circuit (RISC), an application-specific integrated circuit (ASIC), a logic circuit, or any other circuit or processor configured to execute one or more of the functions/methods described herein. In particular, the system for managing sample processing priorities might be integral with a data management system, e.g., implemented on a computing device such as a desktop computer, a laptop, a smartphone, a tablet, etc., may be comprised by a server computer and/or be distributed/shared across/between a plurality of devices. Moreover, the system for managing sample processing priorities can include remote devices, servers and cloud-based elements that communicate via wires or wirelessly (e.g., infrared, cellular, Bluetooth^{®}), or a remote PC/server or a cloud-based system. In particular the system for managing sample processing priority may be integrated in the host, in the at least one sample processing device, or distributed across the host and the at least one sample processing device, e.g. be integrated in a laboratory information system (LIS) and/or a hospital information system (HIS).

The host and the at least one sample processing device can be in communication via a direct connection, wired or wireless, or indirectly over a communications network, wired or wireless, such as a wide area network, e.g., the Internet or a Health Care Provider's local area network or intranet, via a network interface device.

The term "communication network" encompasses also any type of wireless network, such as a WiFi^{™}, GSM^{™}, UMTS, LTE, 5G or other wireless digital network or a cable based network, such as Ethernet^{™} or the like. In particular, the communication network can implement the Internet protocol (IP). For example, the communication network comprises a combination of cable-based and wireless networks.

According to an embodiment, the host system comprises a laboratory information system (LIS) and/or a hospital information system (HIS).

According to an embodiment, the message is in an HL7 or FHIR format.

According to an embodiment, the system for managing sample processing priorities comprises a user interface with an option to select the sample or a sample carrier to be processed with higher priority, and an option to select a change of priority command for the selected sample or sample carrier, in order to change the sample processing priority.

According to an embodiment, the user interface is configured to highlight or mark the selected sample and/or sample processing order and/or sample processing result related to the selected sample in the user interface after a change of priority such as to be distinguishable in a list of samples and/or in of sample processing orders and/or sample processing results.

According to an embodiment, the system for managing sample processing priorities is configured to allow a change of sample processing priority only for routine samples resulting in a change from a routine sample with lower priority to a STAT sample with higher priority, regardless of type of sample carrier.

Other and further objects, features and advantages will appear from the following description of exemplary embodiments and accompanying drawings, which serve to explain the principles more in detail.

### Brief Description of the Drawings

FIG. 1 shows schematically a computer implemented method of managing sample processing priorities.
FIG 2A shows schematically a system for managing sample processing priorities and a first example of managing sample processing priorities according to the method of FIG. 1.
FIG. 2B shows the same system for managing sample processing priorities as in FIG. 2A, but a different use case.
FIG. 2C shows the same system for managing sample processing priorities as in FIG. 2A and

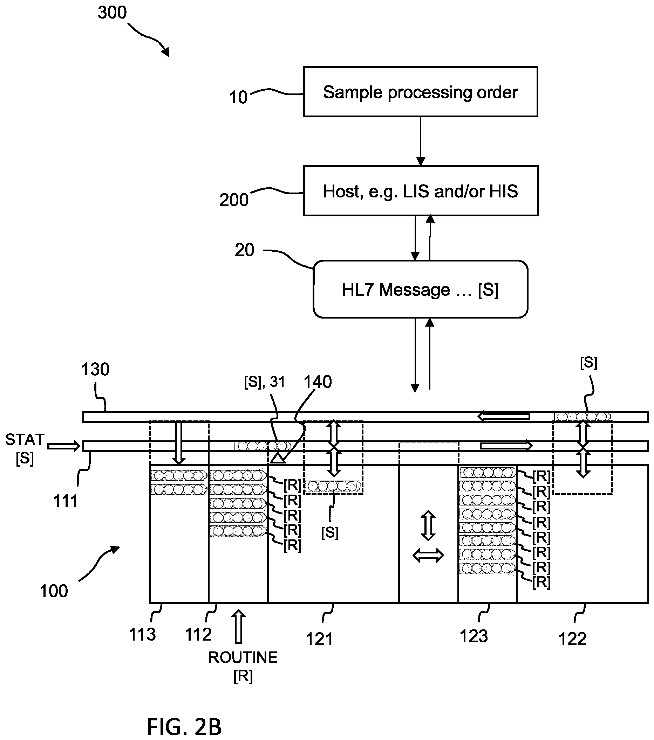

FIG. 2B but yet a different use case.

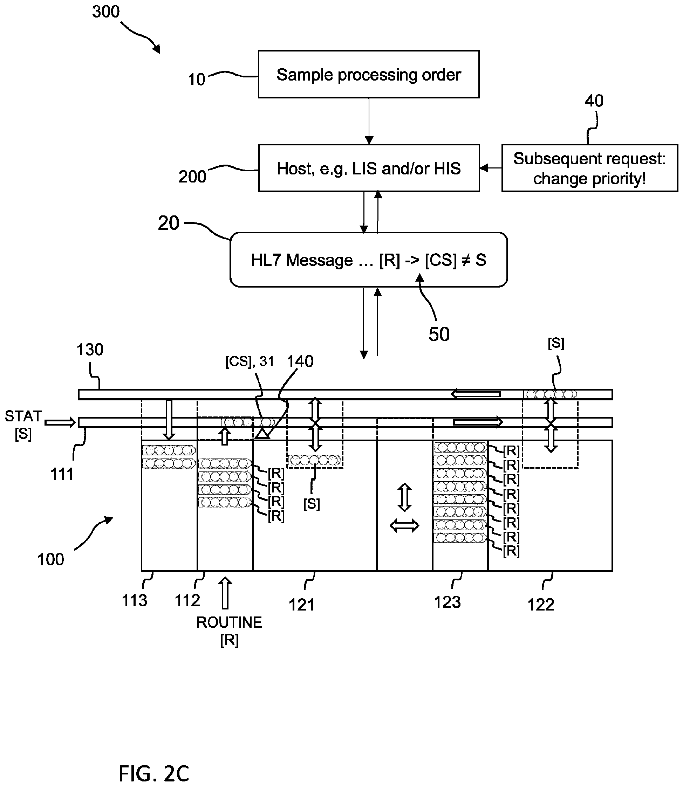

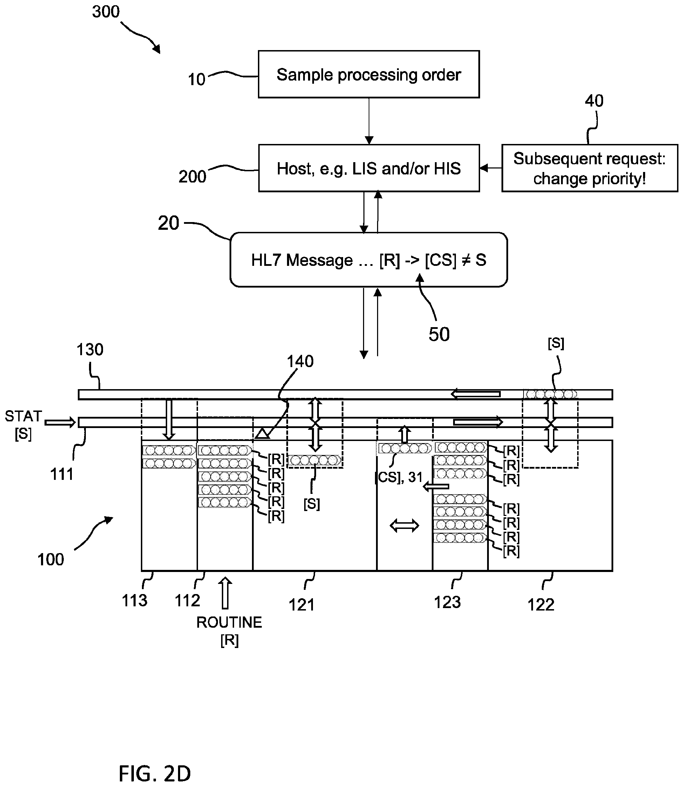

FIG. 2D shows a variant of the case represented in FIG. 2C.

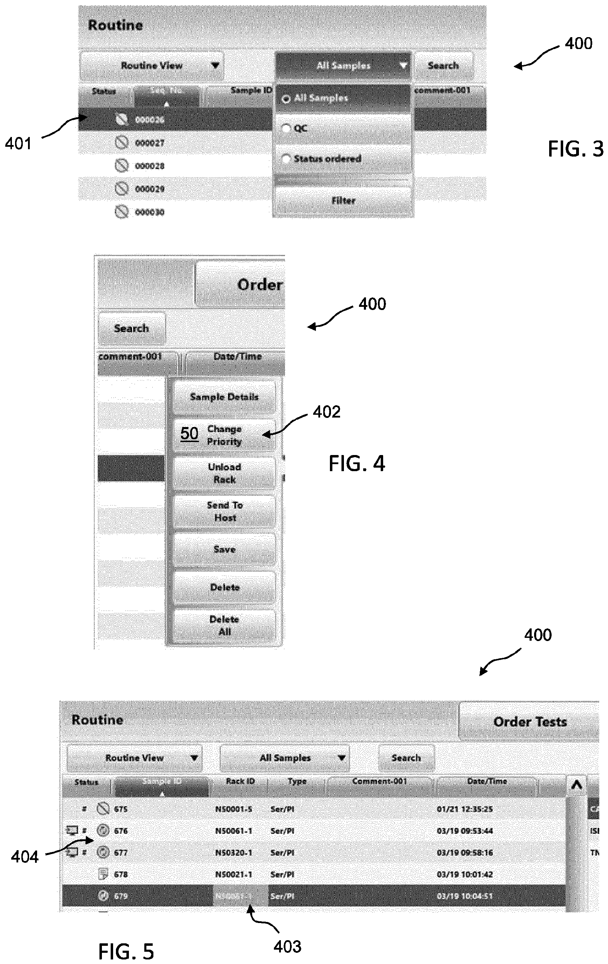

FIG. 3 shows some features of a user interface.
FIG. 4 shows some additional features of the same user interface of FIG. 3.
FIG. 5 shows some additional features of the same user interface of FIG. 3 and FIG. 4.

Skilled artisans appreciate that elements in the figures are illustrated for simplicity and clarity and have not necessarily been drawn to scale. For example, the dimensions of some of the elements in the figures may be exaggerated relative to other elements whereas other elements may have been left out or represented in a reduced number in order to enhance clarity and improve understanding of the embodiments of the present disclosure.

### Detailed Description

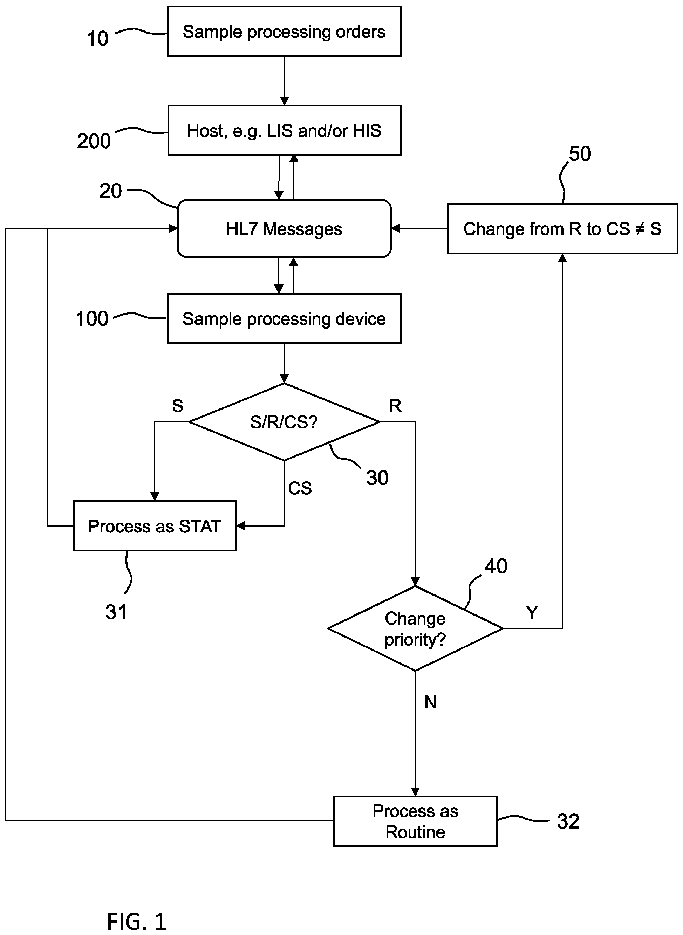

FIG. 1 shows schematically a computer implemented method of managing sample processing priorities in a diagnostic laboratory comprising at least one sample processing device 100 connected to a host system 200. The method comprises generating a communication message 20 between the host 200 and the at least one sample processing device 100 related to a sample processing order 10 received in association with a sample, the message 20 comprising one of at least two priority identifiers R, S indicative of respective sample processing priorities from lower priority (Routine) to higher priority (STAT) according to the received sample processing order 10. The method further comprises identifying the sample by the at least one sample processing device 100, based also on the sample processing priority 30, and processing the sample by the at least one sample processing device 100 according to the sample processing priority 30 in the message 20, in particular processing the sample as a STAT sample 31 if the sample is associated with a higher sample processing priority identifier S or processing the sample as a routine sample 32 if the sample is associated with a lower sample processing priority identifier R. In case of receiving a subsequent request for change 40 of sample processing priority for the sample from a lower priority (Routine) to a higher priority (STAT) after transmission of the message 20 and before the sample is processed, the method comprises changing the message 20 and processing the sample as a higher priority sample 31 instead of as a lower priority sample 32, wherein changing the message comprises changing 50 the lower priority identifier R (routine) to an identifier CS indicative of a change of priority (Changed to STAT) but different from an equivalent higher priority identifier S (STAT) in order to maintain the sample and the sample processing order 10 associated with that sample uniquely identifiable and traceable by both the host system 200 and the at least one sample processing device 100. The host system 200 of this example comprises a laboratory information system (LIS) and/or a hospital information system (HIS). The messages 20 of this example are in a health level seven international (HL7) format.

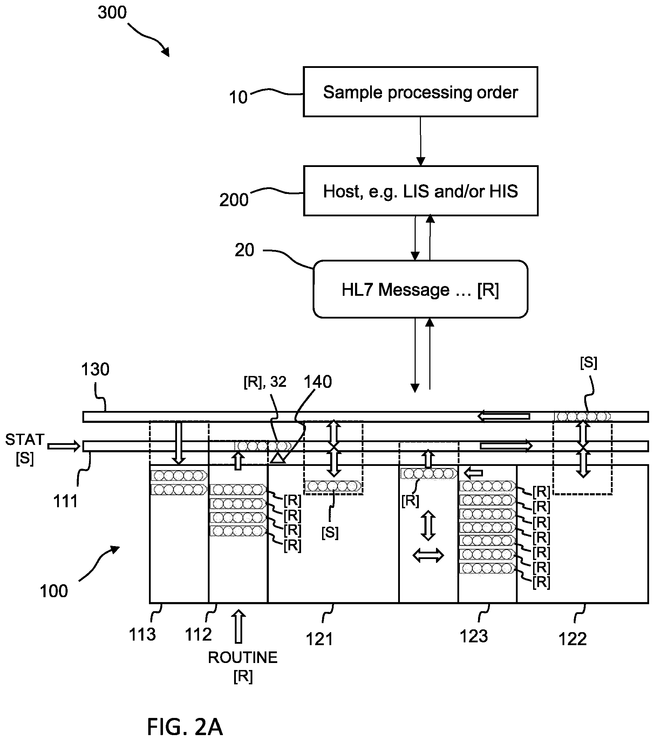

FIG. 2A-2D, when looked at jointly, schematically show a system 300 for managing sample processing priorities in a diagnostic laboratory comprising at least one sample processing device 100 connected to a host system 200, and when looked at individually, schematically show also examples of managing sample processing priorities according to the method of FIG. 1.

In particular, FIG. 2A shows further details and workflow aspects of a sample processing device 100 according to one example. The sample processing device 100 comprises different modules, comprising a STAT sample input port 111 for loading STAT samples with higher sample processing priority [S] in STAT sample carriers, a routine sample input compartment 112 for loading routine samples with lower sample processing priority [R] in routine sample carriers, a sample output compartment 113 for unloading sample carriers of any type after sample processing, a first analytical sample processing module 121 and a second analytical sample processing module 122, a sample buffer compartment 123 for temporarily receiving at least some sample carriers before analytical sample processing by one of the two analytical sample processing modules 121, 122, a sample transportation module 130 for transporting sample carriers within the sample processing device 100. The sample processing device 100 is connected to the host system 200 and they exchange messages 20 related to sample processing orders 10. In this case of FIG. 2A, a sample processing order 10 with lower sample processing priority is received in association with a sample and registered at the host system 200. A message 20 is generated by the host system 200 comprising the priority identifier [R] indicative of lower sample processing priority and communicated to the target sample processing device 100. When the sample arrives at the sample processing device 100, it is loaded via the routine sample input compartment 112 and eventually identified by e.g. a barcode reader 140, and the information contained in the previously received message 20, including the sample processing priority identifier [R], is used to process the sample, in this case as a routine sample 32. In particular, if the sample processing device 100 is already busy in processing other samples and/or other samples are already in a queue and waiting to be processed first, because e.g. arrived first, although they may have the same sample processing priority identifier, or because a STAT sample has arrived soon after, then the sample may be added to the queue, e.g. sent to the sample buffer compartment 123, and may be waiting there for its turn. Otherwise, it may be processed at once, e.g. sent to an available analytical sample processing module 122. Once processed the processing result may be communicated back to the host system 200.

FIG. 2B shows the same system 300 for managing sample processing priorities as in FIG. 2A, but a different use case. In particular, a sample processing order 10 with higher sample processing priority is received in association with a sample and registered at the host system 200. A message 20 is generated by the host system 200 comprising the priority identifier [S] indicative of higher sample processing priority and communicated to the target sample processing device 100. When the sample arrives at the sample processing device 100, it is loaded via the STAT sample input port 111 and identified by e.g. the barcode reader 140, and the information contained in the previously received message 20, including the sample processing priority identifier [S], is used to process the sample, in this case as a STAT sample 31. In particular, samples loaded via the STAT sample input port 111 are given priority with respect to samples loaded via the routine sample input compartment 112 and are identified first. The sample is sent then at once to the first available analytical sample processing module 122 and in any case will be processed before other routine samples that may have arrived first and are already in queue.

FIG. 2C shows the same system 300 for managing sample processing priorities as in FIG. 2A and FIG. 2B but a different use case. In particular, as in the case of FIG. 2A, a sample processing order 10 with lower sample processing priority is received in association with a sample and registered at the host system 200. A message 20 is generated by the host system 200 comprising the priority identifier [R] indicative of lower sample processing priority and transmitted to the target sample processing device 100. When the sample arrives at the sample processing device 100, it is loaded via the routine sample input compartment 112. When the sample is still in the routine sample input compartment 112, thus after transmission of the message 20 and before the sample is processed, a subsequent request for change 40 of sample processing priority is received. In this case, the message 20 is changed by changing 50 the lower priority identifier [R] (Routine) to an identifier [CS] indicative of a change of priority (Changed to STAT) but different from an equivalent higher priority identifier [S] (STAT) in order to maintain the sample and the sample processing order 10 associated with that sample uniquely identifiable and traceable by both the host system 200 and the sample processing device 100. When the sample is eventually identified, by e.g. the barcode reader 140, the information contained in the changed message 20, including the sample processing priority identifier [CS], is used to process the sample. In particular, the sample is processed as a STAT sample 31 with a higher sample processing priority instead of as routine sample 32 with a lower sample processing priority, just as in the case of FIG. 2B.

FIG. 2D shows a case similar to that of FIG. 2C with the difference that a sample had already been previously identified as a routine sample [R] by the sample processing device 100 but is still in queue in the sample buffer compartment 123 when the subsequent request for change 40 of sample priority is received. Also in this case, the message 20 is changed by changing 50 the lower priority identifier [R] (Routine) to an identifier [CS] indicative of a change of priority (Changed to STAT) but different from an equivalent higher priority identifier [S] (STAT) in order to maintain the sample and the sample processing order 10 associated with that sample uniquely identifiable and traceable by both the host system 200 and the sample processing device 100, and the information contained in the changed message 20, including the sample processing priority identifier [CS], is used to process the sample. In particular, the sample that was in a queue, waiting in line for its turn according to a scheduled plan, together with other routine samples, is re-categorized as a STAT sample and given the right and order to skip the line and to be processed as a STAT sample 31, that is earlier than initially scheduled and before other routine samples, e.g. sent at once to the first available analytical sample processing module 122, just as in the case shown in FIG. 2B and FIG. 2C.

FIG. 3 shows an example of user interface 400 with an option 401 to select the sample or a sample carrier to be processed with higher priority.

FIG. 4 shows the same example of user interface 400 of FIG. 3 with an option to select a change of priority command 402 for the selected sample or sample carrier, resulting in the change of the message 50.

FIG. 5 shows the same example of user interface 400 of FIG. 3 and FIG. 4 configured to highlight or mark 403 the selected sample and/or sample processing order and/or sample processing result related to the selected sample in the user interface after a change of priority such as to be distinguishable in a list 404 of samples and/or of sample processing orders and/or sample processing results.

In the preceding specification, numerous specific details are set forth in order to provide a thorough understanding of the present disclosure. It will be apparent, however, to one having ordinary skill in the art that the specific detail need not be employed to practice the present teaching. In other instances, well-known materials or methods have not been described in detail in order to avoid obscuring the present disclosure.

Modifications and variations of the disclosed embodiments are certainly possible in light of the above description. It is therefore to be understood, that within the scope of the appended claims, the invention may be practiced otherwise than as specifically devised in the above examples.

Particularly, it is to be understood that the drawings are only schematic and provided as way of example only. Also the relationship between elements may be other than the one shown. Especially, the identifiers R, S, CS, are arbitrary and any identifiers with the same respective functions and meaning may be in principle used.

Also, reference throughout the preceding specification to "one embodiment", "an embodiment", "one example" or "an example", means that a particular feature, structure or characteristic described in connection with the embodiment or example is included in at least one embodiment. Thus, appearances of the phrases "in one embodiment", "in an embodiment", "one example" or "an example", in various places throughout this specification are not necessarily all referring to the same embodiment or example.

Furthermore, the particular features, structures, or characteristics may be combined in any suitable combinations and / or sub-combinations in one or more embodiments or examples.

## Claims

1. A computer implemented method of managing sample processing priorities in a diagnostic laboratory comprising at least one sample processing device (100) connected to a host system (200), the method comprising
- generating a communication message (20) between the host system (200) and the at least one sample processing device (100) related to a sample processing order (10) received in association with a sample, the message (20) comprising one of at least two priority identifiers (R, S) indicative of respective sample processing priorities from lower priority to higher priority according to the received sample processing order (10),
- identifying (30) the sample by the at least one sample processing device (100) and processing the sample (31, 32) by the at least one sample processing device (100) according to the sample processing priority (S, R) in the message (20), **characterized in that**,
- in case of receiving a subsequent request for change (40) of sample processing priority for the sample from a lower priority to a higher priority after transmission of the message (20) and before the sample is processed, the method comprises changing (50) the message (20) and processing the sample as a higher priority sample (31) instead of as a lower priority sample (32), wherein changing the message (20) comprises changing the lower priority identifier (R) to an identifier (CS) indicative of a change of priority but different from an equivalent higher priority identifier (S) in order to maintain the sample and the sample processing order uniquely identifiable and traceable by both the host system (200) and the at least one sample processing device (100).

2. The method according to claim 1 wherein the host system (200) comprises a laboratory information system (LIS) and/or a hospital information system (HIS).

3. The method according to claim 1 or 2 wherein the message (20) is in a health level seven international (HL7) or Fast Healthcare Interoperability Resources (FHIR) format.

4. The method according to any of the preceding claims wherein changing the message comprises providing a user interface (400) with an option to select the sample (401) or a sample carrier to be processed with higher priority, and an option to select a change of priority command (402) for the selected sample or sample carrier.

5. The method according to claim 4 comprising highlighting or marking (403) the selected sample and/or sample processing order and/or sample processing result related to the selected sample in the user interface (400) after a change of priority such as to be distinguishable in a list (404) of samples and/or of sample processing orders and/or sample processing results.

6. The method according to any of the preceding claims wherein a change (40) of sample processing priority is allowed only for routine samples resulting in a change from a routine sample with lower priority to a short-turnaround-time (STAT) sample with higher priority, regardless of type of sample carrier.

7. A system (300) for managing sample processing priorities in a diagnostic laboratory comprising at least one sample processing device (100) connected to a host system (200), wherein the host system (200) is configured for generating a message (20) between the host system and the at least one sample processing device (100) related to a sample processing order (10) received in association with a sample, the message (20) comprising one of at least two priority identifiers (R, S) indicative of respective sample processing priorities from lower priority to higher priority according to the received sample processing order (10), wherein the at least one sample processing device (100) is configured (140) for identifying the sample and processing the sample (31, 32) according to the sample processing priority (S, R) in the message (20), and wherein the system (300) is further configured for receiving a subsequent request for change (40) of sample processing priority for the sample from a lower priority to a higher priority after transmission of the message (20) and before the sample is processed, the method comprises changing (50) the message (20) and processing the sample as a higher priority sample (31) instead of as a lower priority sample (32), wherein changing the message (20) comprises changing the lower priority identifier (R) to an identifier (CS) indicative of a change of priority but different from an equivalent higher priority identifier (S) in order to maintain the sample and the sample processing order uniquely identifiable and traceable by both the host system (200) and the at least one sample processing device (100).

8. The system (300) according to claim 7 wherein the host system (200) comprises a laboratory information system (LIS) and/or a hospital information system (HIS).

9. The system (300) according to claim 7 or 8 wherein the message (20) is in a health level seven international (HL7) or Fast Healthcare Interoperability Resources (FHIR) format.

10. The system (300) according to any of the claims 7 to 9 comprising a user interface (400) with an option to select the sample (401) or a sample carrier to be processed with higher priority, and an option to select a change of priority command (402) for the selected sample or sample carrier.

11. The system (300) according to claim 10 wherein the user interface (400) is configured to highlight or mark (403) the selected sample and/or sample processing order and/or sample processing result related to the selected sample in the user interface (400) after a change of priority such as to be distinguishable in a list (404) of samples and/or of sample processing orders and/or sample processing results.

12. The system (300) according to any of the claims 7 to 11 wherein the system (300) is configured to allow a change (40) of sample processing priority only for routine samples resulting in a change from a routine sample with lower priority to a short-turn-around-time (STAT) sample with higher priority, regardless of type of sample carrier.
